# EUROPEAN PATENT SPECIFICATION

(11) **EP 2 220 251 B1**
(45) Date of publication and mention of the grant of the patent: **03.08.2016**
(21) Application number: 08838209.8
(22) Date of filing: 10.10.2008
(51) Int. Cl.: C12Q 1/68, G01N 33/68, C07K 14/47, C07K 16/18, A61K 39/395, A01K 67/027, C12N 15/85, C12N 15/11

(54) **METHOD FOR THE DIAGNOSIS OF PATHOLOGIES CHARACTERISED BY THE ANOMALOUS DEPOSITION OF AMYLOID IN ORGANS AND TISSUES BY DETECTING A MUTATION AT POSITION 673 IN APP770, AND VECTOR AND PEPTIDE FOR USE IN THE THERAPY OF SAID PATHOLOGIES**
VERFAHREN ZUR DIAGNOSE VON KRANKHEITEN, DIE DURCH DIE ANOMALE ABLAGERUNG VON AMYLOID IN ORGANEN UND GEWEBEN GEKENNZEICHNET SIND, DURCH DIE BESTIMMUNG EINER MUTATION IN POSITION 673 VON APP770, SOWIE VEKTOR UND PEPTID ZUR THERAPIE SOLCHER KRANKHEITEN
PROCÉDÉ POUR LE DIAGNOSTIC DE PATHOLOGIES, CARACTÉRISÉES PAR LE DÉPÔT ANORMAL DE AMYLOÏDE DANS DES ORGANES ET TISSUS PAR LA DÉTERMINATION D'UNE MUTATION À POSITION 673 DE LA PROTEINE APP670, ET VECTEUR ET PEPTIDE POUR LA THERAPIE DE LAQUELLES PATHOLOGIES

(30) Priority: 12.10.2007 IT MI20071975
(43) Date of publication of application: 25.08.2010
(73) Proprietor: Fondazione I.R.C.C.S. Istituto Neurologico 'Carlo Besta', 20133 Milano (IT)
(72) Inventor: DI FEDE, Giuseppe, I-85028 Rionero in Vulture (IT); MORBIN, Michela, I-20133 Milano (IT); TAGLIAVINI, Fabrizio, I-20131 Milano (IT); MARTINI, Alfredo, I-20145 Milano (IT)
(74) Representative: Rapisardi, Mariacristina
(86) International application number: PCT/EP2008/008595
(87) International publication number: WO 2009/047002

(56) References cited:
- WO-A-2004/058940
- WO-A-2006/121656
- WO-A-2007/064917
- WO-A2-2004/099376
- DATABASE EBI [Online] EMBL; 26 February 2004 (2004-02-26), DIAS NETO E. ET AL: "RC0-HT0505-010200-012-h02 HT0505 Homo sapiens cDNA, mRNA sequence" XP002528966 Database accession no. BG877527
- PEACOCK M L ET AL: "Novel polymorphism, in the A4 region of the amyloid precursor protein gene in a patient without Alzheimer's disease" NEUROLOGY, LIPPINCOTT WILLIAMS & WILKINS, PHILADELPHIA, US, vol. 43, no. 6, 1 January 1993 (1993-01-01), pages 1254-1256, XP009093023 ISSN: 0028-3878
- JONES C T ET AL: "Mutation in codon 713 of the beta amyloid precursor protein gene presenting with schizophrenia." NATURE GENETICS JUL 1992, vol. 1, no. 4, July 1992 (1992-07), pages 306-309, XP002528905 ISSN: 1061-4036
- TUGYI REGINA ET AL: "Partial D-amino acid substitution: Improved enzymatic stability and preserved Ab recognition of a MUC2 epitope peptide" PROCEEDINGS OF THE NATIONAL ACADEMY OF SCIENCES OF THE UNITED STATES OF AMERICA, vol. 102, no. 2, 11 January 2005 (2005-01-11), pages 413-418, XP002529045 ISSN: 0027-8424
- DI FEDE GIUSEPPE ET AL: "A recessive mutation in the APP gene with dominant-negative effect on amyloidogenesis." SCIENCE (NEW YORK, N.Y.) 13 MAR 2009, vol. 323, no. 5920, 13 March 2009 (2009-03-13), pages 1473-1477, XP002528904 ISSN: 1095-9203

## Description

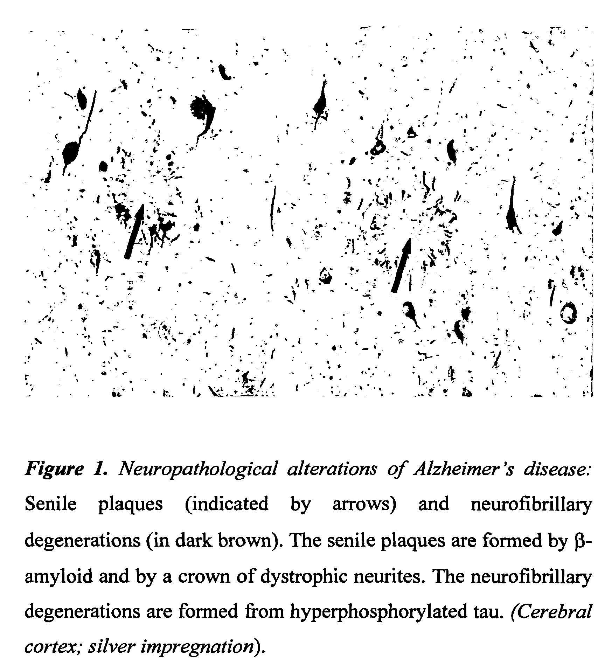

Alzheimer's disease is the most common form of dementia in elderly people. It is a degenerative disease clinically characterised by the progressive decline of cognitive functions, and neuropathologically characterised by the accumulation of insoluble aggregates of β-amyloid (Aβ) and tau protein, in the cerebral cortex and in subcortical grey matter. The Aβ is deposited in the form of extracellular amyloid in the neuropile (senile plaque) and in the cerebral vessels (congophilic angiopathy), while the tau form of the protein forms anomalous intraneuronal filaments (neurofibrillary degenerations) (Love S. Neuropathological investigation of dementia: a guide for neurologists. J Neurol Neurosurg Psychiatry 76, Suppl 5:v8-14, 2005) (Figure 1).

In 95% of the cases, Alzheimer's disease is sporadic, while in about 5% of the cases it has a familial character and is associated with mutations of 3 genes: presenilin 1 (PSEN 1) on chromosome 14, presenilin 2 (PSEN2) on chromosome 1 and precursor of the β-amyloid (APP) on chromosome 21. In these cases, the disease often has an earlier onset than the sporadic form and is transmitted with a mechanism of autosomal dominant type with high penetration. AD's ethiopathogenesis is not yet entirely understood, but in the last decade the hypothesis has been increasingly confirmed of the "amyloid cascade" (Wilquet et al. Amyloid-beta precursor protein processing in neurodegeneration. Curr Opin Neurobiol 14:582-8, 2004; Lee et al. Perspectives on the amyloid-beta cascade hypothesis. J Alzheimers Dis 6:137-45, 2004) which attributes a central role to the Aβ both in the familial (FAD) and sporadic forms.

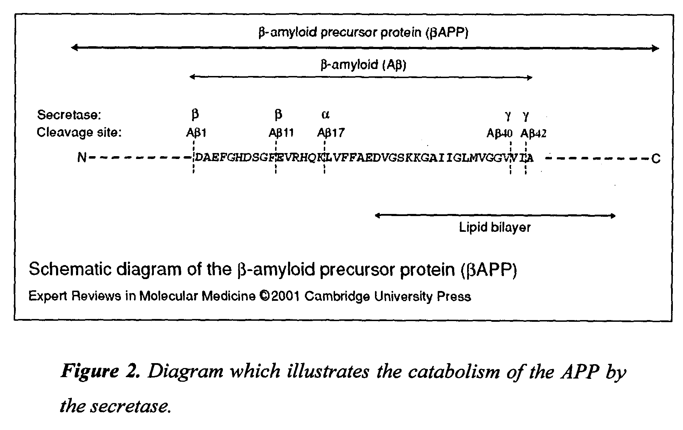

The Aβ derives from its βAPP precursor through a catabolic pathway called "amyloidogenic pathway" (Figure 2). This pathway provides for the cleavage of the molecule upstream and downstream of the β-protein by two proteases, the beta-secretase and the gamma-secretase. The cutting of the beta-secretase (BACE) generates a long, soluble N-terminal fragment (sAPPβ) and a C-terminal peptide of 99 amino acids (C99). This is further cut by the gamma-secretase into two fragments which correspond to Aβ and a small C-terminal peptide (AICD) (Selkoe DJ. Deciphering the genesis and fate of amyloid β-protein yields novel therapies for Alzheimer disease. J Clin Invest 110:1375-81, 2002). The gamma-secretase, in reality, has two main cleavage sites which lead to the formation of a "short" and a "long" form of Aβ (Aβ1-40 and Aβ1-42), which in normal conditions have a ratio of 10:1. Analogously, BACE can act at different points of the peptide, generating truncated forms in the N-terminal region (for example, Aβ11-40, Aβ11-42 and Aβ3-42), which often result increased in AD (Liu et al. Characterization of Aβ11-40/42 peptide deposition in Alzheimer's disease and young Down's syndrome brains: implication of Alzheimer's disease. Acta Neuropathol 112:163-74, 2006). βAPP can encounter an alternative catabolic pathway called "non-amyloidogenic", since the protein is cut by another protease (alpha-secretase) at the residues 16-17 of Aβ. The action of the latter enzyme thus precludes the formation of β-amyloid.

The amyloid cascade hypothesis is supported by multiple proofs:
- mutations of the gene APP determine familial forms of AD (Rademakers et al., Genetics of Early-Onset Alzheimer Dementia. ScientificWorldJournal 16:497-519, 2003);
- the presence of an extra copy of the gene APP, as is verified in Down's syndrome, suffices for determining a clinical-pathological description of AD;
- most of the genetically determined forms of AD are associated with an increase of the Aβ production, with an increase of the ratio Aβ42/Aβ40 (Kahle et al. Attack on amyloid. EMBO Rep 4:747-51, 2003);
- Aβ, particularly in the "long" 42-residue form, shows a strong tendency to be aggregated in oligomers and to form amyloid fibrils which represent the main constituent of the senile plaques (Armstrong RA. Plaques and tangles and the pathogenesis of Alzheimer's disease. Folia Neuropathol 44:1-11,2006);
- Aβ, especially the 42 amino acid form, is neurotoxic (Butterfield et al. Amyloid beta-peptide (1-42) contributes the oxidative stress and neurodegeneration found in Alzheimer disease brain. Brain Pathol 14:426-32, 2004);
- transgenic mice, carriers of the APP gene associated with AD, accumulate β-amyloid in the central nervous system and show deficits of the behavioural-cognitive sphere which are worsened as a function of age (Kurt et al. Neurodegenerative changes associated with beta-amyloid deposition in the brains of mice carrying mutant amyloid precursor protein and mutant presenilin-1 transgenes Exp Neurol 171:59-71, 2001);
- the immunisation towards Aβ of transgenic mice expressing human APP reduces the formation of amyloid plaques and improves its neurological deficits (Lemere et al. Amyloid-beta immunization in Alzheimer's disease transgenic mouse models and wildtype mice. Neurochem Res 28:1017-27, 2003).

Regarding the genetically determined forms, about 80% of the familial AD cases are associated with PSEN1 and PSEN2 mutations (Rocchi et al. Causative and susceptibility genes for Alzheimer's disease: a review. Brain Res Bull 61:1-24, 2003). Both presenilins are involved in the generation of Aβ, being part of the macromolecular complex of the gamma-secretase, and their mutations case an increase of the Aβ production, above all of Aβ1-42, which has a high tendency to form neurotoxic aggregates. About 5% of the FAD are caused by mutations localised on the APP gene (Rocchi et al. Causative and susceptibility genes for Alzheimer's disease: a review. Brain Res Bull 61:1-24, 2003) (Figure 2). Some of these mutations exert their pathogenic effect by favouring conformations of Aβ rich in secondary beta-sheet structure, with consequent reduction of the solubility and tendency towards aggregation. Other mutations, on the other hand, would interfere with the processing of the APP, due to their localisation in the sites of the molecule where the secretases act (for example, "Swedish mutation" KM670/671NL). Still others, with entirely unknown mechanism, cause the production accumulation of long and insoluble forms of Aβ (Aβ1-42 and Aβ1-43). In the AD cases associated with mutations of the APP or of the presenilins, the Aβ1-42 increases until it constitutes 15-40% f the secreted Aβ peptides, while in normal conditions it represents only 5-10% thereof (Rocchi et al. Causative and susceptibility genes for Alzheimer's disease: a review. Brain Res Bull 61:1-24, 2003; Lleò et al. Clinical, Pathological, and Biochemical Spectrum of Alzheimer Disease Associated with PS-1 Mutations. Am J Geriatr Psychiatry 12:146-56. 2004).

The entry in DATABASE EBI [Online] EMBL; 26 February 2004 (2004-02-26), DIAS NETO E. ET AL: "RC0-HT0505-010200-012-h02 HT0505 Homo sapiens cDNA, mRNA sequence" Database accession no. BG877527 discloses a sequence which contains a C to T polymorphism at a position which corresponds to residue 2212 of the APP770 gene. WO 2004/099376 discloses expression vectors with mutated beta-secretase cleavage sites wherein one of the vectors contain the sequence NLDV which differs from the Swedish isoform of APP only in the A673V substitution.

The technical task of the present invention is that of providing methods for the diagnosis of and products for use in the prevention and/or care of human and/or animal pathologies characterised by the anomalous deposition of β-amyloid substance and/or amyloid-like substance in human and/or animal tissues and/or organs, and a screening method for determining the risk of such pathologies.

The technical task, as well as other objects according to the present invention, are achieved by means of that revealed in the independent claims reported below.

Other characteristics of the invention are defined by the subsequent claims.

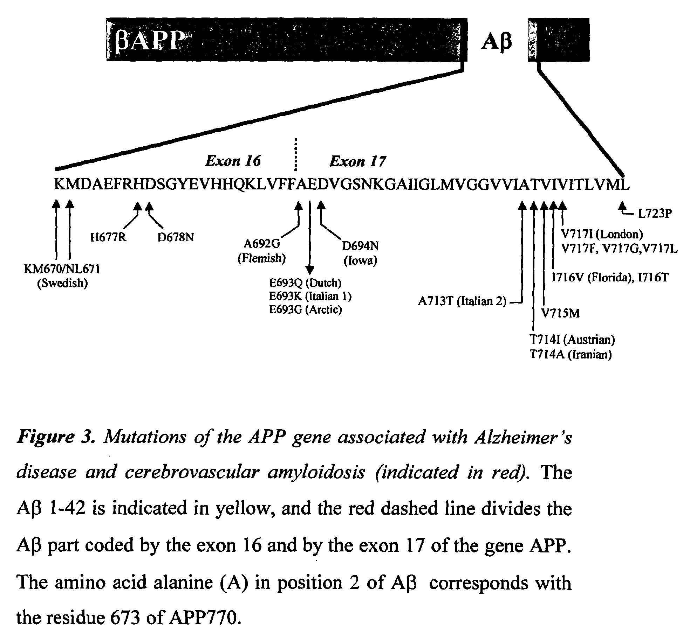

Further characteristics and advantages of the present invention are more evident from the following description supported by the attached figures 1-19.

The present invention refers to the recent discovery of a new punctiform mutation of the human APP gene. The mutation is characterised by the substitution of a Cytosine with a Thymidine at codon 673 of the coding sequence of the human APP gene (D8765), corresponding with the nucleotide 2212 (transition *c.2212>T*) of the isoform of human APP770 (*NM*_*000484*.*2*) according to the nomenclature of the *GenBank* database, accessibly on the website http://www.ncbi.nlm.nih.gov. For the purposes of this patent, by amyloid-like substances, it is intended protein aggregates of Aβ which do not have the tinctorial and/or ultrastructural characteristics of the amyloid itself. Such mutation, which induces in the protein sequence the substitution of an alanine with a valine in position 673 (Ala673Val) of APP770, corresponding with the amino acid residue 2 of Aβ, was identified in homozygosis of a patient affected with a grave form of dementia with presenile onset. The analysis of the cephalorachidian liquid of the patient showed a considerable diminution of the total tau protein and phosphorylated tau, as is observed in Alzheimer disease. On the other hand, the plasma levels of Aβ1-40 and Aβ1-42 are increased with respect to control subjects and also with respect to subjects that bear the same mutation in heterozygosis. In addition, the fibroblasts obtained from skin biopsy of the patient released, in their culture medium, higher quantities of Aβ1-40 and Aβ1-42 with respect to control fibroblasts. Overall, this data, whose details are reported in several of the examples listed below, indicates that the mutation Ala673Val, in homozygosis state, is associated with a dementia that can be described as Alzheimer's disease, and, analogous to other mutations of the APP gene, influences the processing of the APP by increasing the Aβ production.

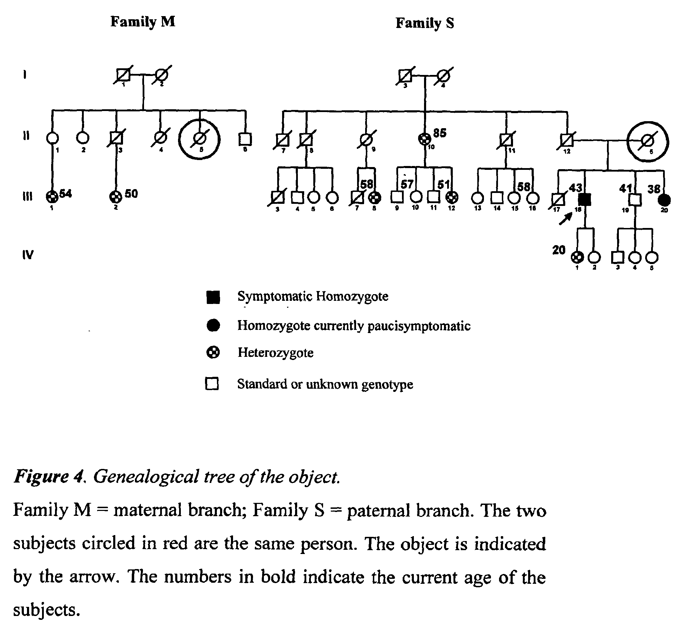

The genetic study of different family members demonstrated the presence of another familiar carrier of the Ala673Val mutation in homozygosis. This relative, younger than the patient, was subjected to neuropsychological evaluation, which detected initial signs of compromise of different cognitive functions. Genetic analysis permitted, moreover, identifying numerous carrier subjects of the same mutation in heterozygosis which, surprisingly, had developed no neurological development, even if some of them were advanced in age (IX decade of life) (Figure 4). Gene expression studies carried out on the transcribed RNA starting from the gene APP demonstrated that in these subjects, both the alleles (i.e. wild type and mutated) are transcribed. Therefore, the absence of disease in the heterozygotes cannot be due to a gene repression mechanism (inhibition of the transcription of the "pathological" allele). It can therefore be hypothesized that the Ala673Val mutation, contrary to that described up to now in the APP gene, all autosomal dominant with complete penetration, has an expression of autosomal recessive type. It follows that several apparently sporadic forms of AD could be genetically caused with autosomal recessive transmission.

In order to investigate the molecular bases of this phenomenon, we have synthesised 2 Aβ1-40 peptides, one wild-type (*DAEFRHDSGYEVHHQKLVFFAEDVGSNKGAIIGLMVGGVV*), the other containing a valine in place of the alanine in position 2 (*DVEFRHDSGYEVHHQKLVFFAEDVGSNKGAIIGLMVGGVV*).

The two peptides were subjected to chemical-physical and morphological analysis aimed to evaluate their secondary structure, the aggregation kinetics, and the morphology and nature of the aggregates. These investigations showed that the mutated peptide had a propensity to form amyloid fibrils that were much larger than the wild-type. Quite surprisingly, the mixture composed of equimolar quantities of the two peptides not only aggregates less than the mutated peptide but also less than the wild type peptide on its own. This "inhibitory" effect on the amyloidogenesis coincides with the clinical observation that the disease is exclusively manifested in the homozygote subjects for the Ala673Val mutation while the heterozygotes, which co-express both the peptides at the cellular level (wild-type and mutated), do not fall ill. On the basis of this data, it can even be assumed that the heterozygote individuals can be protected from Alzheimer's disease due to the small fibrillogenic tendency of the mutated Aβ peptide in the presence of its corresponding wild-type.

In order to verify the hypothesis that the N-terminal region of Aβ, which houses the mutation, plays an important rule in the aggregation and that the Ala673Val mutation has an inhibitory effect, two peptides were synthesised corresponding with the first six amino acids of Aβ, one with the wild-type sequence (DAEFRH) and the other containing a valine in place of the alanine in position 2 (DVEFRH). The two hexapeptides were then co-incubated with the Aβ1-40 wild-type and examined at subsequent times. The study demonstrated that both the hexapeptides inhibit the spontaneous tendency for the fibrillogenesis of Aβ1-40 and that the effect of the mutated hexapeptide is greater than that of the corresponding wild-type.

This data opens new possibilities in the scope of therapeutic strategies for AD, and more in general of the diseases characterised by protein accumulation in the form of insoluble and toxic aggregates in the central nervous system or in other tissues.

A first application of our disclosure consists of the production, according to methods known by those skilled in the art, of a vector containing the cDNA of the human APP with Ala673Val, and the use of said vector in order to transfect cell lines usable for pathogenesis studies and therapy.

A second application consists of the use of the construct according to the previous application as vector for the production, according to methods known by those skilled in the art, of transgenic non-human mammals capable of expressing human APP with Ala673Val mutation, as single form of APP (homozygote animals) or in combination with wild type human APP or containing another mutation (double transgenic). Such animals can be used as models for the study of pathogenesis, diagnosis, prevention and care of human and/or animal pathologies, characterised by the anomalous formation and deposition of β-amyloid and/or amyloid-like substance in organs and tissues. In the current embodiment, the preferred animal is the mouse, and in particular the knockout murine strain C57BL6 for the endogenous APP, and the preferred pathology is AD.

Considering the potential capacity of the mutated peptide to interfere with the aggregation and the fibrillogenesis of Aβ, another possible application of our invention is represented by the generation of a construct containing APP with Ala673Val mutation in the in vivo gene therapy (the DNA is transferred directly in the cells or tissues of the patient) or ex vivo gene therapy (the DNA is first transferred in cells isolated from the organism and laboratory-grown, which, thus modified, can be re-introduced in the patient) of pathologies characterised by anomalous deposition of β-amyloid substance in tissues and organs. The transfer of the construct into the target cells can be achieved by means of vectors of viral type, such as for example (a) retroviruses which have the capacity to integrate their DNA inside the proliferation cell chromosomes, (b) lentiviruses which allow transferring genetic material also in cells which do not proliferate, (c) adeno-associated viruses which do not integrate their DNA in the chromosomes of the cell but can be used only for genes of small size, (d) adenoviruses which can transport genes of large size but nevertheless ensure their expression for limited time periods, or (e) herpex simplex virus which only infects several types of cells, in particular the neurons. Alternatively, it is possible to use non-viral vectors like the liposomes. The introduction of APP with Ala673Val mutation in organisms affected by pathologies with abnormal accumulations of Aβ could provide a source of mutated β-protein capable of inhibiting the accumulation of β-amyloid substances in the tissues.

Another possible application is represented by the use of negative-sense mRNA, containing the present mutation, for inhibiting the translation of the messenger in homozygote subjects for the Ala673Val mutation, according to methods known to those skilled in the art (RNA interference, RNAi). The inhibition of the translation has the object of causing a block of the production of the mutated peptides, which have the strong tendency towards aggregation. The experiments based on RNAi technology applied to our disclosure can also be useful in the study of the pathogenesis of diseases characterised by anomalous formation and deposition of β-amyloid and/or amyloid-like substances in the tissues and organs. Another application of our disclosure provides for the use of the human APP with Ala673Val mutation and natural or synthesis peptides containing the mutation itself for the diagnosis, prevention and care of human and/or animal pathologies, characterised by anomalous formation and deposition of β-amyloid and/or amyloid-like substances in the tissues and organs.

Our preferred embodiment provides for the use of low molecular weight peptides, like the hexapeptide DVEFRH, suitably formulated for the oral and/or parenteral administration, including the intrathecal administration. The preferred pathology is AD. The treatment provides for the administration of single peptides or the association of several peptides, used as single treatment or in association with other drugs.

A further application of our disclosure provides for the production, by means of techniques known to those skilled in the art, of antibodies towards the proteins and/or peptides pursuant to the previous application, to be used in the diagnosis, prevention and/or care of the of human and/or animal pathologies, characterised by anomalous formation and deposition of β-amyloid and/or amyloid-like substances in the tissues and organs.

The disclosure provides for a monoclonal antibody capable of recognising the Ala673Val mutation in the human APP and in peptides derived therefrom and containing such mutation. Such antibody can be used for diagnostic purposes in order to recognise the APP with Ala673Val mutation or, suitably formulated, for the treatment of amyloidosis characterised by the presence of this mutated APP. The preferred amyloidosis is AD.

The applications described above are reported as an example, and are not in any manner limiting of the developments of our invention which is defined by the claims.

### EXAMPLES

### Example 1: Identification of a new mutation of the APP gene and description of the clinical phenotype of the carrier patient of such mutation.

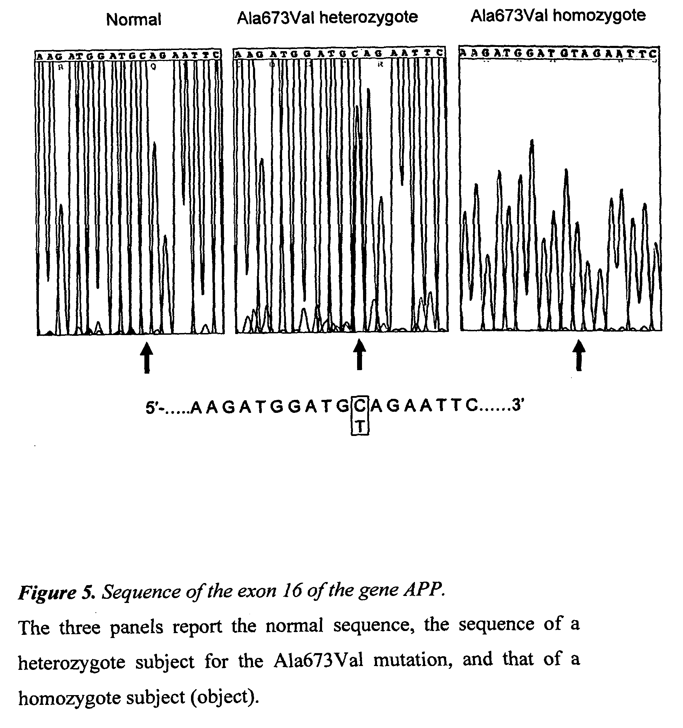

The identification of the mutation was conducted by means of the extraction of the genome DNA from the patient lymphocytes, amplification of the exons 16 and 17 of the gene APP by means of polymerase chain reaction (PCR), using the primers 5'-GTTTTGGGTAGCCTTTG-3 and 5'-GGCAAGACAAAACAGTAGTGG-3' and sequencing of the amplification product (Figure 5) according to already described techniques (Wakutani et al. Novel amyloid precursor protein gene missense mutation (D678N) in probably familial Alzheimer's disease. J Neurol Neurosurg Psychiatry 75:1039-42, 2004).

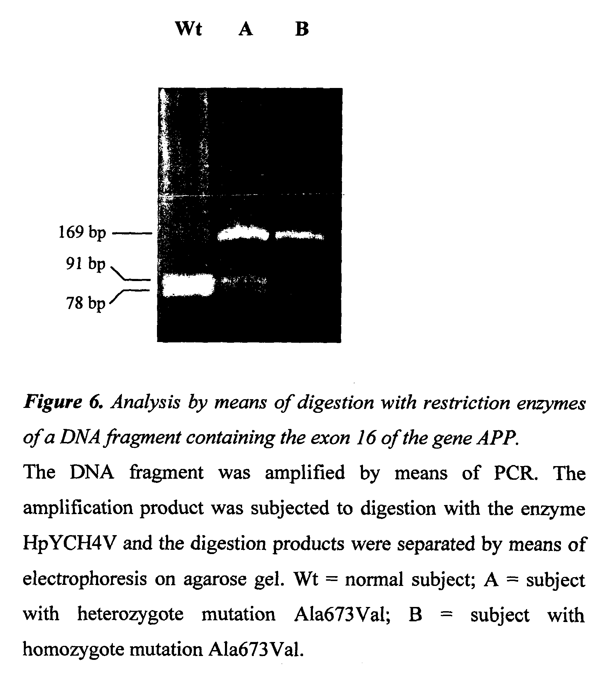

Since the mutation eliminates a specific cutting site for the restriction enzyme HpYCH4V inside the exon 16, the presence of Ala673Val is also shown by means of amplification of the exon 16 through PCR (primers: 5'-GGCAAGACAAAACAGTAGTGG-3' and 5'-TACTTTAATTATGATGTAATA-3'), digestion of the PCR product with HpYCH4V, and separation of the fragments on 2.5% agarose gel. In the wild type allele, the digestion with HpYCH4 produces two fragments of 91 and 78 base pairs (bp), while the mutated allele generates a single fragment of 169 bp (Figure 6).

The Ala673Val mutation was identified in homozygosis in a patient without familiality for dementia, affected by an evolutive psycho-organic syndrome with onset at age 36, with ingravescent memory deficits, planning difficulties and behavioural disturbances (Figure 4, III 18). The clinical description evolved towards a serious multi-sector cognitive decay, to which involuntary movements are associated of myoclonic type, Parkinsonism and spastic tetraparesis.

The genetic study of the family allowed identifying a second homozygote subject for the Ala673Val mutation (Figure 4, III 20) and different heterozygote subjects (Figure 4, II 10, III 1, III 2, III 8, III 12, IV 1). The homozygote (i.e. the patient's sister, five years younger) currently has initial signs of cognitive deterioration compatible with an onset of the disease; on the other hand, none of the heterozygote subjects have shown signs of neurological pathology, not even in advanced age. This observation suggests that the Ala673Val mutation is autosomal recessive, resulting in that it is the only one of those described up to now in association with AD that expresses a pathological phenotype only when present in homozygosis.

It should be underlined that the same codon of the APP gene houses an Ala673Thr polymorphism. This polymorphism was encountered in heterozygosis in a subject without clinical signs or neuropathological alterations suggestive of AD (Peacock et al. Novel polymorphism in the A4 region of the amyloid precursor protein gene in a patient without Alzheimer's disease. Neurology 43:1254-56, 1993).

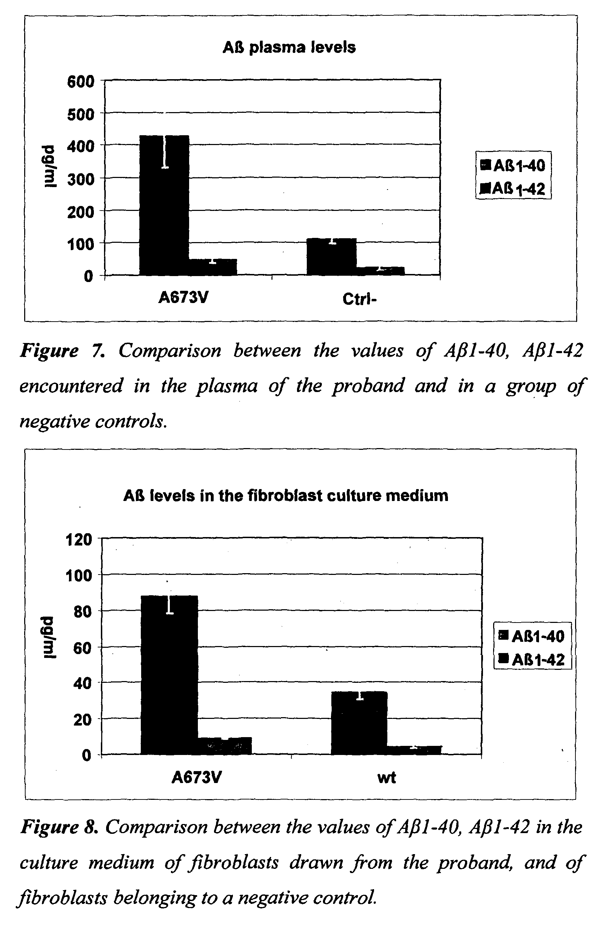

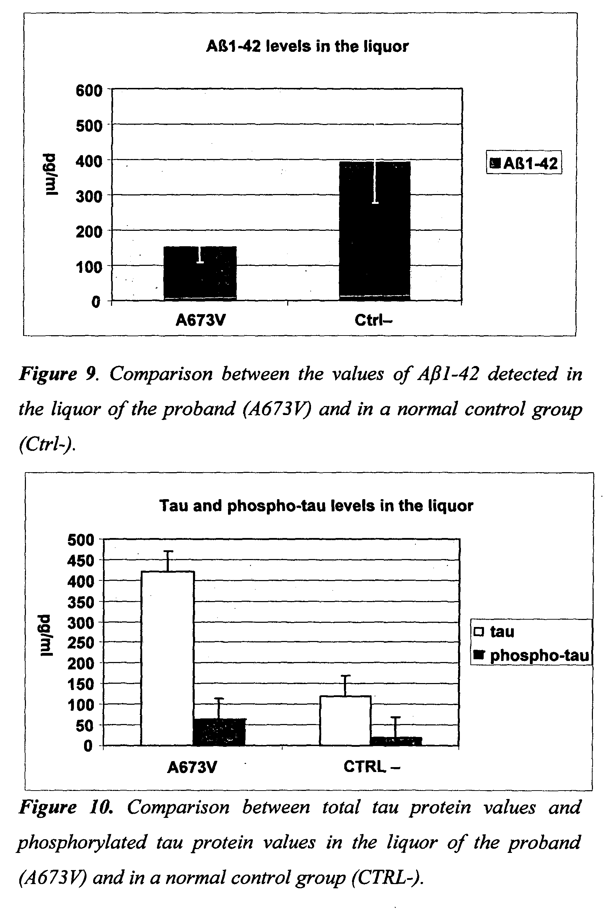

The laboratory and instrument research carried out on the patient showed:
- widespread cerebral atrophy, with prevalent involvement of the front regions, at the RM of the encephalon;
- significant increase of the peptides Aβ1-40 and Aβ1-42 in the plasma (426 ± 93 pg/ml and 46 ± 7 pg/ml, respectively) compared with a control group represented by subjects not affected by dementia (Aβ1-40 = 109 ± 12 pg/ml, p=0.003; Aβ1-42 = 20 ± 6 pg/ml, p=0.004) (Figure 7);
- increase of Aβ in the culture medium of the fibroblasts drawn from the patient by means of skin biopsy (Aβ1-40 = 87.3 ± 9.5 pg/ml; Aβ1-42 = 8.8 ± 0.2 pg/ml) with respect to the negative controls (Aβ1-40 = 34.4 ± 3.8 pg/ml; Aβ1-42 = 4.4 ± 0.6 pg/ml) (Figure 8);
- decrease of Aβ1-42 ± 43 pg/ml versus 392 ± 115 pg/ml of a control group, p=0.0004) (Figure 9), and increase of the tau protein (420 pg/ml; normality range 90-150 pg/ml) and phosphor-tau (63.3 pg/ml; average concentration in the controls: 19.1 pg/ml) (Figure 10) in the cerebral-spinal liquid.

The alterations described are entirely similar to those observed in Alzheimer's disease.

### Example 2: Analysis of the chemical physical characteristics of Aβ peptides containing the Ala673Val mutation.

In order to ascertain the effects of the Ala673Val mutation and verify its role in the pathogenesis of AD, we synthesised 2 Aβ-40 peptides, one with the wild-type (DAEFRHDSGYEVHHQKLVFFAEDVGSNKGAIIGLMVGGV V) sequence and the other containing alanine>valine in position 2 (DVEFRHDSGYEVHHQKLVFFAEDVGSNKGAIIGLMVGGV V). The peptides were produced by means of solid phase synthesis by using a synthesiser 433A (Applied Biosystems). The peptides bonded to the resin were then derivatised at the N-terminal with a lipophile probe (4-dodecylaminocarbonylfluorene-9-ylmethylsuccinimidyl carbonate) according to the method described by Ball et al. (Int J Pept Prot Res 40:370-9, 1992) with the modifications introduced by Bonetto et al. (J Biol Chem 277: 31327-34, 2002). After separation from the resin, the peptides were purified by means of HPLC, by using a reverse phase column C4 (Waters), obtaining a purity >95%. The identity of the peptides was determined by means of MALDI-TOF spectrometry (Reflex III Brucker Model).

For the physical-chemical studies reported below (unless otherwise specified), the peptides wild-type Aβ1-40, mutated Aβ1-40 and samples containing equimolar mixtures of the two were dissolved in 10 mM NaOH and subsequently diluted in 50 mM Tris HCl, pH 7.0, at the final concentration of 0.25 and 0.125 mM. The samples were then incubated at 37°C for 1, 4, 8, 24 hours and 3, 5, 10, 15 and 20 days. For each time, aliquots of the samples were analysed in order to determine the secondary structure, the aggregation, the ultrastructure and the optical-tinctorial properties of the aggregate.

### Secondary structure

The variations induced by the mutation of the secondary structure of Aβ were investigated by means of Circular Dichroism according to the technique described by Clippingdale et al. (J Pept Sci 5:227-49, 2001). The peptides were diluted in 150 mM phosphate buffer, pH 7.4, to the final concentration of 100 µM, and the measurements were conducted with a Jasco-810 spectropolarimeter at a constant 37°C temperature. The spectra were acquired by using a 1 mm test tube and a scanning speed of 20 nm/min. After having obtained the spectrum of the buffer solution, the noise was reduced, when required, by using the moving average method.

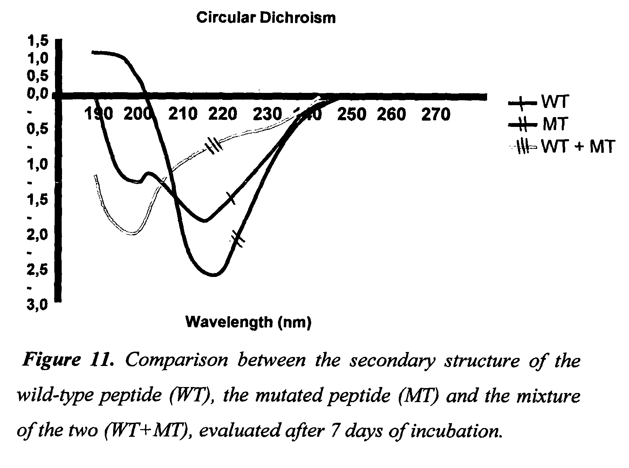

The analysis demonstrates that the mutated peptide had a strong tendency to assume a secondary conformation abounding in β-sheets. At all examined times, the β-sheet content was much higher not only with respect to that of the wild-type peptide but also with respect to that of the equimolar mixture constituted by mutated wild-type peptides (Figure 11).

This indicates that the Ala673Val mutation conditions the folding of Aβ, causing a considerable increase of the secondary β-sheet structure.

### Aggregation

The aggregation of wild-type Aβ1-40, mutated Aβ1-40 and their equimolar mixture was evaluated by determining the quantity of peptide that could be sedimented with centrifugation. At the different incubation times, 30 µl aliquots of the samples were centrifuged at 15,000 g for 15 minutes at 4°C. The pellet was solubilised in 25 µl of pure formic acid, and the solution was injected in HPLC provided with PRLP-S 100Å column, 4.6 x 150 mm (Labservice Analytica, Polymer Laboratories). The elution was made by using as movable phase an eluent A composed of 0.1% TFA in water and an eluent B constituted by 0.08% TFA in acetonitrile, at a flow speed of 0.7 ml/min, applying a 15-60% linear gradient of the eluent B in 20 min. The peak corresponding to the peptide was modified by measuring the absorbance of the eluate at 214 nm.

The quantity of peptide that can be sedimented was calculated as percentage of the total quantity of peptide present in the initial solution.

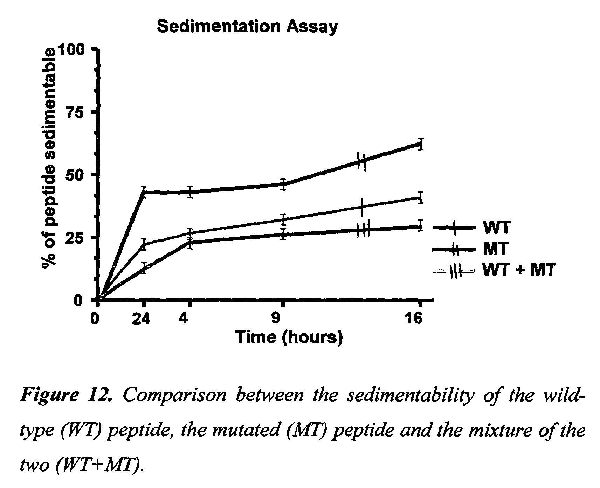

These experiments demonstrated that the mutated peptide aggregated much more and much more quickly than the wild-type peptide and that, surprisingly, the mixture formed by the two peptides sediments less than the mutated peptide as well as the wild-type peptide (Figure 12).

### Ultrastructure and tinctorial properties of the aggregates

The ultrastructural characteristics and optical-tinctorial properties of the aggregates were respectively studied by means of electronic microscope and polarised light microscope after coloration with Congo Red.

For the ultrastructural investigation, 5 µl of suspension of wild-type Aβ1-40, mutated Aβ1-40 and their equimolar mixture were drawn at incubation times in the range of 1 hour - 20 days, deposited on nickel screen covered with Formvar-Carbon for 5 minutes, negatively coloured with an over-saturated solution of uranyl and observed under electronic microscope (EM109 Zeiss). On the twentieth day of incubation, aliquots of the samples were centrifuged at 15,000 g for 15 minutes. The pellets thus obtained were fixed in 2.5% glutaraldehyde in phosphate buffer, pH 7.4, post-fixed in 1% osmium tetroxide, dehydrated in acetone and included in epoxy rein (Spurr, Electron Microscopy Sciences). Ultrafine sections (500 Å) were collected on copper screens, coloured with uranyl acetate and lead citrate and observed under the electronic microscope.

In order to verify if and in what measure the aggregates were constituted by amyloid, 5 µl of solution of each sample, for the different incubation times, was collected on polylysinated slides (Bio-Optical), coloured with Congo Red and examined with polarised light microscope (Nikon Eclipse E-800).

The ultrastructural analysis showed that in the first two days of incubation, the wild-type Aβ1-40 peptide forms amorphous aggregates, oligomers and rare filamentous structures. After 48 hours, a short fibril material appears, not ramified, irregular (protofibril), and only after 72 hours of incubation are long rectilinear fibrils observed, of about 8 nm diameters, interposed with amorphous and protofibril material. Subsequently, the density of the fibrils increases and the quantity of amorphous and protofibril material is proportionally increased. Only after 15 days of incubation is most of the material composed of dense fibril networks.

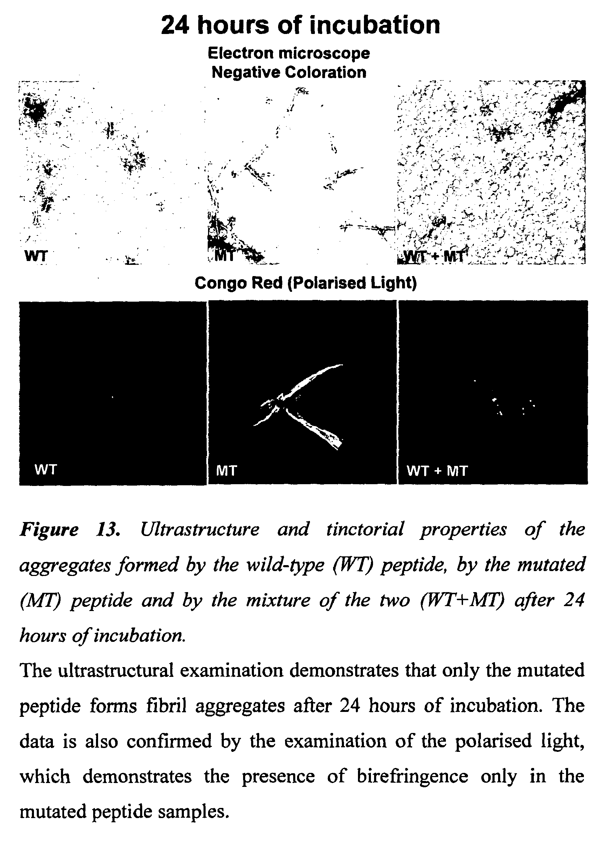

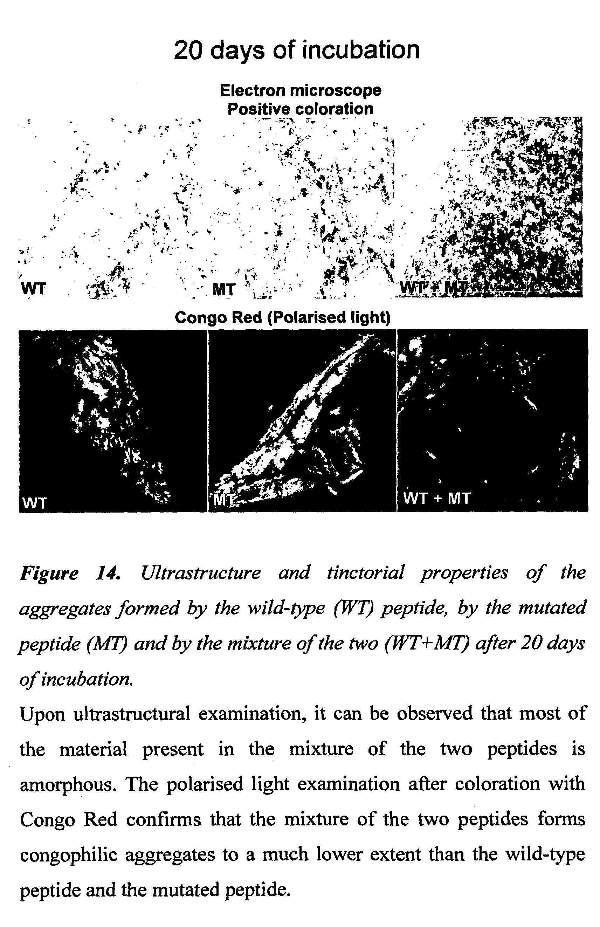

On the other hand, the aggregation kinetics of the mutated peptide Aβ1-40 were very fast. Indeed, starting from 24 hours of incubation, long, regular fibrils lacking ramifications were present (Figure 13), and after 5 days the sample was constituted by dense fibril networks, without protofibrils and amorphous material. Surprising, the equimolar mixture of the two peptides forms less fibrils not only with respect to the mutated peptide, but also with respect to the wild-type, and after 20 days of incubation most of the aggregates were composed of amorphous material (Figure 14). The observation in polarised light of the preparations coloured with Congo Red showed that the mutated peptide Aβ1-40 is much more amyloidogenic than wild-type Aβ1-40 and that the mixture of the two peptides has a low tendency to form amyloid. In fact, small aggregates of birefringent material were already present after 24 hours of incubation (Figure 13) in the mutated Aβ1-40 samples, after 72 hours in the wild-type Aβ1-40 samples and only after 5 days in the mixture of the two peptides. At later time, a progressive increase of birefringent material was observable in the mutated Aβ1-40 and wild-type Aβ1-40 samples, while the increase was very small in the mixture of the two peptides, even after 20 days of incubation (Figure 14).

This data confirms the results of the aggregation studies, demonstrating that (i) the mutated peptide Aβ1-40 is much more amyloidogenic than the wild-type, and (ii) the mixture of the two peptides has a low tendency to form amyloid fibrils.

### Example 3. Inhibition of the amyloidogenesis by means of synthetic peptides, homologues of the N-terminal region of Aβ containing the Ala673Val mutation.

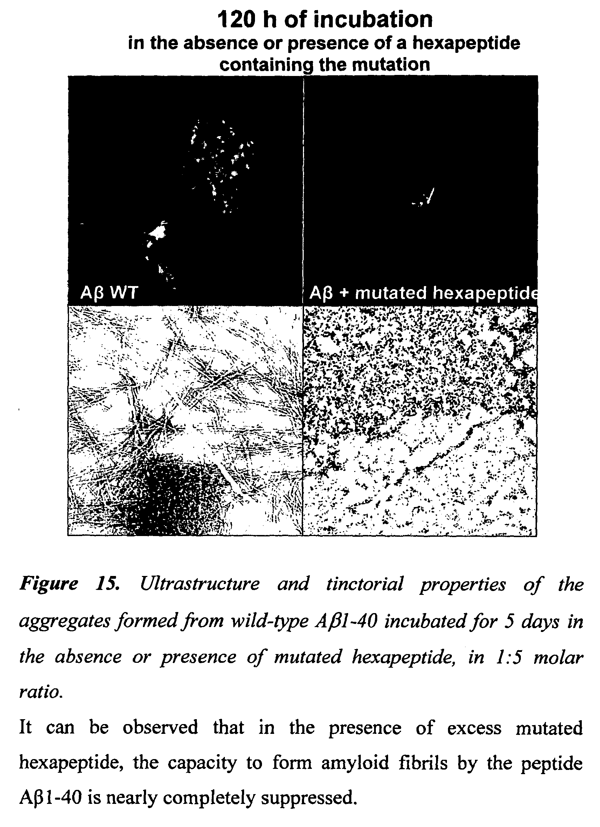

Since the physical-chemical study of the mixture of the mutated Aβ1-40 and wild-type Aβ1-40 peptides suggested that the Ala673Val mutation could have an inhibitory effect on the aggregation of Aβ, we verified this hypothesis by using two synthetic peptides corresponding to the first six amino acids of Aβ, one with the wild-type sequence (DAEFRH) and the other containing a valine in place of the alanine in position 2 (DVEFRH). The two hexapeptides were co-incubated with wild-type Aβ1-40 at equimolar concentration or in excess (hexapeptide:Aβ1-40 = 5:1). The mixtures were prepared for the ultrastructural and histochemical study as described in example 2. The study has shown that both the hexapeptides (both the mutated and the wild-type) inhibit the fibrillogenesis of Aβ1-40, indicating that the N-terminal region of Aβ, site of the mutation, plays an important role in the aggregation (Figure 15). Nevertheless, the mutated hexapeptide resulted more active than the corresponding wild-type, underlining the importance of the Ala673Val mutation due to the inhibitory effect on the fibrillogenesis.

### Example 4: Transfection of cell lines with wild-type human APP, or containing the Ala> Val mutation in position 2 of Aβ.

By means of genetic engineering methods (Tesco et al. APP substitutions V715F and L720P alter PS 1 conformation and differentially affect Aβ and AICD generation. J Neurochem 95: 446-56, 2005; Sudhir et al. Release of Amino-terminal Fragments from Amyloid Precursor Protein Reporter and Mutated Derivatives in Cultured Cell. J Biol Chem 267:25602-08, 1992) two vectors were generated respectively containing the cDNA of wild-type human APP751 and the cDNA of human APP751 with the Ala>Val mutation in position 2 of Aβ. With these vectors, two cell lines were transfected (COS7 and CHO), on which Aβ metering was carried out in the medium with ELISA method.

The Ala>Val mutation in position 2 of Aβ was inserted in the cDNA of human APP751 by means of site-specific mutagenesis (QuikChange® XL Site-Directed Mutagenesis Kit, Stratagene) using the oligonucleotides 5'-GATCTCTGAAGTGAAGATGGATGTAGAATTCC-3' and 5'-GTCATGTCGGAATTCTACATCCATCTTCACTT 3'. Both the wild-type and mutated form of APP were then amplified by means of PCT, by using the primers 5'-CCCGGATATCGCCACCATGCTGCCCGGTTTGGCAC-3' and 5'-ACCGAAGCTTTGTGGCGGGGGTCTAGTTC-3' (the first containing a site recognised by the restriction enzyme EcoRV, the second with site for the enzyme HindIII), and cloned in the vector pcDNA 3.1, at the restriction sites EcoRV and HindIII. The constructs thus produced were further amplified by means of transformation of Top Ten One Shot (Invitrogen) cells, purified by means of the kit Endofree Plasmid Maxi Kit (Qiagen), and used for transfecting COS7 and CHO cells by means of electroporation. The efficiency of the transfections was evaluated through the quantification of APP on cell lysates by means of Western blot, using the antibody 22C 11 (Chemicon International Inc.) directed against the N-terminal region of the protein (residues 61-88). The APP expression level was used for comparing the levels of Aβ production by cells transfected with two constructs. On the culture medium of the COS7 and the CHO expressing wild-type and mutated human APP, the metering was then carried out of peptides Aβ1-40, Aβ1-42 and truncated forms at the N-terminal with ELISA (Immuno-Biological Laboratories Gunma).

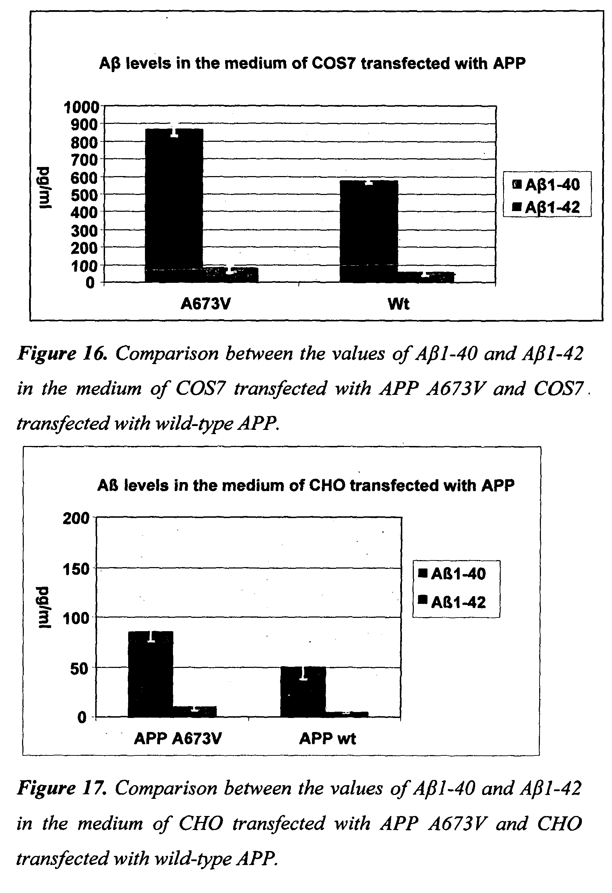

The study demonstrated:
- a strong increase of Aβ1-40 and Aβ1-42 in the medium of the COS7 cells transfected with mutated APP (116.8 ± 90.5 pg/ml and 20 ± 12.3 pg/ml, respectively) with respect to cells transfected with wild-type APP (21.9 ± 8.6 pg/ml and 4 ± 0.8 pg/ml) (Figure 16);
- a strong increase of Aβ1-40 and Aβ1-42 in the medium of CHO transfected with mutated APP (84.6 ± 9 pg/ml and 9.6 ± 3.4 pg/ml, respectively) with respect to cells transfected with wild-type APP (49.8 ± 11.8 pg/ml and 4.2 ± 0.8 pg/ml) (Figure 17);
- a significant increase of the truncated forms at the N-terminal of Aβ, in particular Aβ3-42, in the medium of the COS7 cells transfected with mutated APP (2.5 ± 0.3 pg/ml) with respect to cells transfected with wild-type APP (1.1 ± 0.3 pg/ml).

This date indicates that the Ala>Val mutation in position 2 of Aβ modifies the processing of APP, favouring the amyloidogenic pathway, with increase of production of Aβ1-40, Aβ1-42 and truncated forms at the N-terminal.

### Example 5: Generation of transgenic mice, carriers of the Ala>Val mutation in position 2 of Aβ.

We made a construct carrying human APP with Ala>Val mutation in position 2 of Aβ, for the generation of transgenic mice on which behavioural, neurophysiological, neuroradiological, neuropathological, biochemical and molecular tests were conducted in order to define the phenotype characteristics of the disease associated with this genetic defect, and to conduct pathogenesis and therapy studies.

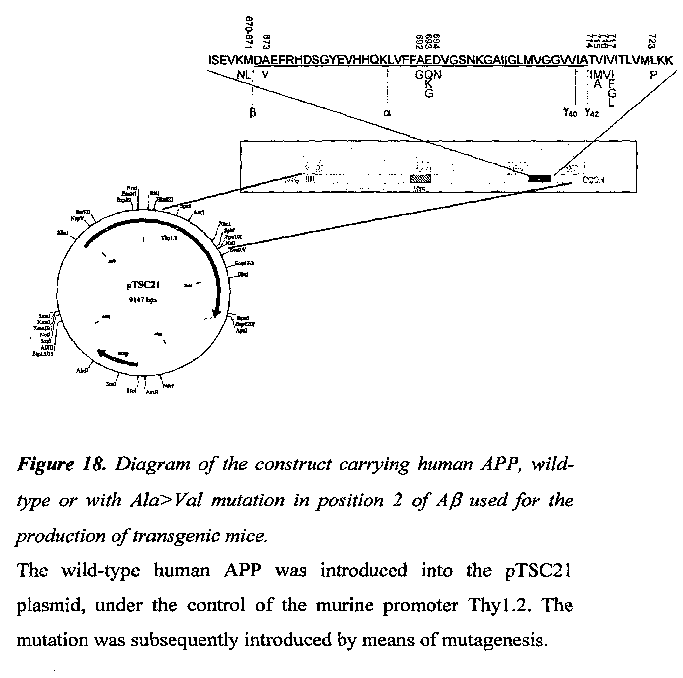

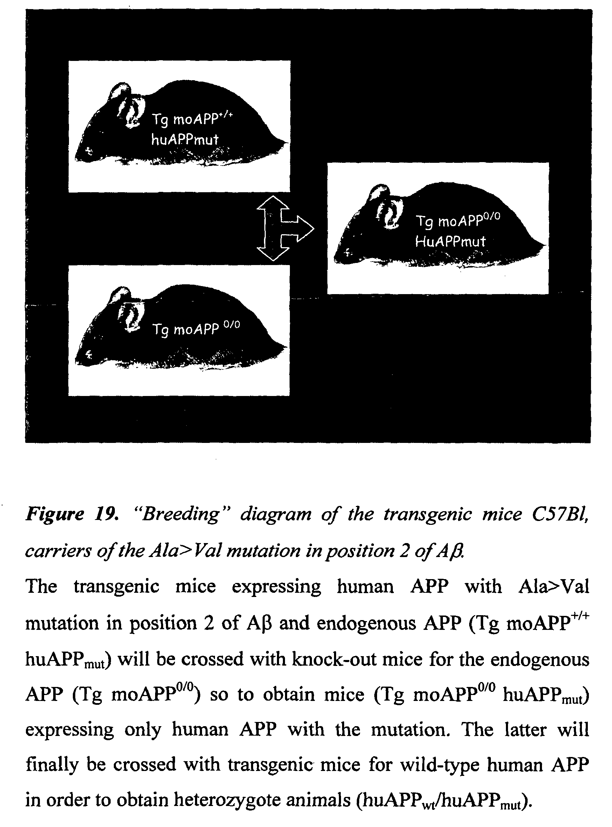

The cDNA of wild-type APP751 was cloned in the vector pTSC21, containing the promoter murine Thy 1.2 (restriction sites HindIII and EcoRV) (Figure 18). The construct was then subjected to site-specific mutagenesis with insertion of the Ala>Val mutation in position 2 of Aβ (Stratagene) by means of the same protocol reported for the cell transfections (see Example 4), and it was used for generating transgenic mice starting from the strain *G57Bl*/*6*. 6 founders (3 male and 3 female) positive for the transgene were obtained, which gave life to three lines which over-express human APP with Ala>Val mutation in position 2 of Aβ in the central nervous system. The two best lines will be crossed with a line of C57B1/6 knock-out mice for endogenous APP - line already available - in order to obtain animals expressing mutated human APP in the absence of murine APP (huAPPₘᵤₜ/moAPP^{0/0}, Figure 19). Finally, these will be crossed with transgenic mice for wild-type human APP in order to obtain heterozygote animals (huAPPₘᵤₜ/huAPP_{wt}).

The mice expressing human APP with mutation 2 of Aβ in homozygosis and heterozygosis will be used for pathogenesis studies, diagnosis, prevention and care of Alzheimer's disease and, more in general, of human and/or animal diseases characterised by an anomalous deposition of amyloid and/or amyloid-like substance in organs and tissues.

### GLOSSARY

AD = Alzheimer's disease
AICD = C-terminal fragments which derives from the cutting of APP by the γ-secretase
APP = Protein precursor of β-amyloid
APP_{0/0} = Knock-out animal for APP endogenous
APP_{673A} = APP wild-type
APP₆₇₃ᵥ = APP with Ala>Val mutation at codon 673
Aβ = β-amyloid, peptide deriving from the catabolism of APP
BACE = β-secretase
bp = base pair
COS Cells = kidney cells of adult male Cercopithecus aethiops transformed with a defective mutant of the SV40 virus
Cellule CHO = Cells derived from Chinese hamster ovary
DHPLC = Denaturing high performance liquid chromatography
DNA = deoxy-ribonucleic acid
FAD = Familial form of Alzheimer's disease
HPLC = High performance liquid chromatography
huAPP = normal human APP
huAPPₘᵤₜ = transgenic mice expressing human APP with Ala>Val mutation in position 2 of Aβ
huAPP_{wt} = transgenic mice expressing wild-type human APP
MAPT = gene coding for the tau protein
moAPP = murine APP
moAPP^{+/+ =} mice with normal APP expression
moAPP^{0/0} = knock-out mice for the endogenous APP
mRNA = messenger RNA
Mut = mutated
P_{CMV} = Promoter of Cytomegalovirus
PCR = Polymerase chain reaction
PSEN1 = Presenilin 1
PSEN2 = Presenilin 2
RM = Magnetic resonance
RNA = ribonucleic acid
RNAi = RNA interference
sAPPβ = Soluble fragment which derives from the cutting of APP by the β-secretase
SSCP = single strand conformation polymorphism
Wt = wild-type

## Claims

1. *In vitro* screening method carried out on human biological material for determining the risk of pathologies **characterised by** anomalous deposition of β-amyloid and/or amyloid-like substance formed by any isoform of Aβ, based on the determination of the substitution, in homozygote or heterozygote form, of a cytosine with a thymidine at codon 673 of the sequence coding the human APP gene (D87675), corresponding with the nucleotide 2212 (c.2212C>T transition) of the isoform of APP770 (NM_000484.2), the mutation resulting in the substitution of Alanine with Valine at the residue 673 of APP770, or at the analogous residue of other isoforms of APP, which correspond to the position 2 of Aβ.

2. *In vitro* screening method according to claim 1, based on the research of messenger RNA (mRNA) transcribed by the gene.

3. *In vitro* screening method according to claim 1, based on the research of the protein APP and/or its isoforms.

4. *In vitro* screening method according to claims 1-3, **characterized in that** one of said pathologies is the Alzheimer's disease in its typical form or expressed in atypical phenotypes.

5. Vector carrying nucleic acids which encode an isoforms of human APP or a fragment containing the Ala673Val mutation, under the control of a non-endogenous promoter, for use in the somatic gene therapy of human and/or animal pathologies **characterized by** the anomalous deposition of β-amyloid substance and/or amyloid-like substance in human and/or animal tissues and organs.

6. Vector for use according to claim 5, wherein the somatic therapy is achieved by using as carrier vectors, natural or synthetic, Vector for use according to claim 7, wherein the somatic lipids or polymers, or biological agents including viral agents, optionally Adenovirus, Adeno-associatedvirus, SV40 virus or retrovirus.

7. Vector for use pursuant to claim 5 for the transfection of autologous, heterologous or xenologous cells for use in the cell therapy of sporadic or genetic forms of Alzheimer's disease with typical or atypical phenotype.

8. Synthetic hexapeptide DVEFRH containing at least one amino acid residue in dextrorotatory form for use in the treatment of pathologies **characterized by** the anomalous deposition of β-amyloid substance and/or amyloid-like substance in human and/or animal tissues and organs.

9. synthetic peptides for use in the treatment of pathologies **characterized by** the anomalous deposition of β-amyloid substance and/or amyloid-like substance in human and/or animal tissues and organs, whereby the synthetic peptide contains at least one amino acid residue in dextrorotatory form, is analogous to a fragment of APP including isoforms of Aβ including those truncated at the N-terminal and/or extended at the C-terminal and comprises the Ala673Val mutation.

## Patentansprüche

1. *In-vitro*-Screeningverfahren, durchgeführt bei menschlichem biologischem Material zum Nachweis des Risikos von Krankheiten, die durch die anomale Ablagerung einer β-Amyloid- und/oder amyloidähnlichen Substanz gekennzeichnet sind, gebildet durch eine beliebige Isoform von Aβ, basierend auf dem Nachweis der Substitution, in homozygoter oder heterozygoter Form, eines Zytosins mit einem Thymidin am Codon 673 der Sequenz, das für das menschliche Gen APP codiert (D87675), entsprechend dem Nukleotid 2212 (Transition c.2212C>T) der Isoform APP770 (NM_000484.2), wobei die Mutation zur Substitution von Alanin mit Valin am Rest 673 von APP770 führt, oder am analogen Rest anderer Isoformen von APP, welche der Position 2 von Aβ entsprechen.

2. *In*-*vitro*-Screeningverfahren nach Anspruch 1, basierend auf der Untersuchung nach Messenger-RNA (mRNA), die vom Gen transkribiert wird.

3. *In*-*vitro*-Screeningverfahren nach Anspruch 1, basierend auf der Untersuchung nach dem Protein APP und/oder seiner Isoformen.

4. *In*-*vitro*-Screeningverfahren nach den Ansprüchen 1 bis 3, **dadurch gekennzeichnet, dass** eine der Krankheiten die Alzheimer-Krankheit in ihrer typischen Form ist oder in atypischen Phänotypen exprimiert ist.

5. Vektor, Nukleinsäuren tragend, die für eine Isoform menschlicher APP oder eines Fragments, enthaltend die Ala673Val-Mutation, unter der Kontrolle eines nicht-endogenen Promotors codieren, zur Verwendung bei der somatischen Gentherapie menschlicher und/oder tierischer Krankheiten, die durch die anomale Ablagerung einer β-Amyloid-Substanz und/oder amyloidähnlichen Substanz in menschlichen und/oder tierischen Geweben und Organen gekennzeichnet sind.

6. Vektor zur Verwendung nach Anspruch 5, wobei die somatische Therapie durch Verwendung als Trägervektoren, von natürlichen oder synthetischen Lipiden oder Polymeren, oder biologischen Wirkstoffen, einschließlich viralen Erregern, wahlweise Adenovirus, Adeno-assoziierten Virus, SV40-Virus oder Retrovirus, erzielt wird.

7. Vektor zur Verwendung nach Anspruch 5 zur Transfektion von autologen, heterologen oder xenologen Zellen zur Verwendung in der Zelltherapie von sporadischen oder genetischen Formen der Alzheimer-Krankheit mit typischem oder atypischem Phänotyp.

8. Synthetisches Hexapeptid DVEFRH, enthaltend mindestens einen Aminosäurerest in rechtsdrehender Form zur Verwendung bei der Behandlung von Krankheiten, die durch die anomale Ablagerung einer β-Amyloid-Substanz und/oder amyloidähnlichen Substanz in menschlichen und/oder tierischen Geweben und Organen gekennzeichnet sind.

9. Synthetische Peptide zur Verwendung bei der Behandlung von Krankheiten, die durch die anomale Ablagerung einer β-Amyloid-Substanz und/oder amyloidähnlichen Substanz in menschlichen und/oder tierischen Geweben und Organen gekennzeichnet sind, wobei das synthetische Peptid mindestens einen Aminosäurerest in rechtsdrehender Form enthält, analog zu einem Fragment von APP einschließlich von Isoformen von Aβ einschließlich solcher, die am N-Terminus verkürzt sind und/oder am C-Terminus verlängert sind, ist, und die Ala673Val-Mutation umfasst.

## Revendications

1. Procédé de criblage *in vitro* mené sur matériel biologique humain pour déterminer le risque de pathologies **caractérisées par** le dépôt anormal de β-amyloïde et/ou d'une substance analogue à l'amyloïde formée par une quelconque isoforme Aβ, basé sur la détermination de la substitution, sous forme homozygote ou hétérozygote, d'une cytosine par une thymidine à codon 673 de la séquence codant pour le gêne humain APP (D87675), correspondant au nucléotide 2212 (transition c.2212 C>T) de l'isoforme APP770 (NM_000484.2), la mutation produisant la substitution d'Alanine par Valine au résidu 673 d'APP770, ou au résidu analogue d'autres isoformes APP, qui correspondent à la position 2 d'Aβ.

2. Procédé de criblage *in vitro* selon la revendication 1, basé sur la recherche d'ARN messager (ARNm) transcrit par le gène.

3. Procédé de criblage *in vitro* selon la revendication 1, basé sur la recherche de la protéine APP et/ou ses isoformes.

4. Procédé de criblage *in vitro* selon les revendications 1 à 3, **caractérisé en ce que** l'une desdites pathologies est la maladie d'Alzheimer dans sa forme typique ou exprimée par des phénotypes atypiques.

5. Vecteur portant des acides nucléiques codant pour une isoforme de la protéine humaine APP ou un fragment contenant la mutation Ala673Val, sous le contrôle d'un promoteur non endogène, en vue de son utilisation dans la thérapie génique somatique de pathologies humaines et/ou animales, **caractérisées par** le dépôt anormal de la substance β-amyloide et/ou d'une substance analogue à l'amyloïde dans les tissus et organes humains et/ou animaux.

6. Vecteur en vue de son utilisation selon la revendication 5, dans lequel la thérapie somatique consiste en l'utilisation, en tant que vecteurs porteurs, de lipides ou polymères naturels ou synthétiques, ou d'agents biologiques, incluant des agents viraux, soit Adénovirus, Adénovirus associés, virus SV40 ou rétrovirus.

7. Vecteur en vue de son utilisation selon la revendication 5 pour la transfection de cellules autologues, hétérologues ou xénologues à utiliser dans la thérapie cellulaire de formes sporadiques ou génétiques de la maladie d'Alzheimer avec phénotype typique ou atypique.

8. Hexapeptide synthétique DVEFRH contenant au moins un résidu d'acide aminé sous forme dextrogyre, en vue de son utilisation dans le traitement de pathologies **caractérisées par** le dépôt anormal de la substance β-amyloïde et/ou d'une substance analogue à l'amyloïde dans les tissus et organes humains et/ou animaux.

9. Peptides synthétiques en vue de leur utilisation dans le traitement de pathologies **caractérisées par** le dépôt anormal de la substance β-amyloide et/ou d'une substance analogue à l'amyloïde dans les tissus et organes humains et/ou animaux, dans lesquels le peptide synthétique contient au moins un résidu d'acide aminé sous forme dextrogyre, est analogue à un fragment d'APP incluant des isoformes Aβ, y compris celles tronquées en N-terminal et/ou allongées en C-terminal et comprend la mutation Ala673Val.
